# EUROPEAN PATENT SPECIFICATION

(11) **EP 1 859 832 B1**
(45) Date of publication and mention of the grant of the patent: **26.05.2010**
(21) Application number: 06725809.5
(22) Date of filing: 09.03.2006
(51) Int. Cl.: A61M 25/02

(54) **DEVICE FOR FIXING CANNULAS AND SIMILAR INSTRUMENTS TO A PART OF THE HUMAN BODY**
VORRICHTUNG ZUR FIXIERUNG VON KANÜLEN UND ÄHNLICHEN INSTRUMENTEN AN EINEM TEIL EINES MENSCHLICHEN KÖRPERS
DISPOSITIF DE FIXATION DE CANULES ET ANALOGUES A UNE PARTIE DU CORPS HUMAIN

(30) Priority: 16.03.2005 ES 200500614
(43) Date of publication of application: 28.11.2007
(73) Proprietor: Sabater Robles, José, E-30110 Cabezo De Torres Murcia (ES)
(72) Inventor: Sabater Robles, José, E-30110 Cabezo De Torres Murcia (ES)
(74) Representative: Carpintero Lopez, Francisco
(86) International application number: PCT/ES2006/000111
(87) International publication number: WO 2006/097553

(56) References cited:
- EP-A- 0 245 754
- WO-A1-98/10823
- WO-A2-93/00788
- ES-U- 270 292
- US-A- 3 834 380
- US-A- 4 275 721
- US-A- 4 490 141
- US-A- 4 490 141
- US-A- 4 534 762
- US-A- 5 546 938

## Description

### OBJECT OF THE INVENTION

The present invention relates to a device for fixing cannulas and the like to a part of the human body. Such a device is shown in the WO 9 810 823.

### FIELD OF THE INVENTION

The field of the invention is clinical therapeutics, in particular patient care requiring the intravenous application of medication, nutritional fluids and the like. The new device will be used preferably by specialised medical practitioner and be compatible with any type of other therapeutic material used in hospitals and clinics.

### BACKGROUND OF THE INVENTION

Currently, the application of therapeutic fluids to patients presents various problems associated to the difficulty of maintaining cannulas, catheters and similar elements used for the immediate transfer of products from their containers to the human body in question in their functional position.

There are different means for holding a cannula, catheter or other element for therapeutic transfusion in a temporarily unmovable way in its position of operative use, ranging from a simple strip of plaster to the use of clamps and similar elements. Fixing is often precarious due to the natural movements of the patient, the fixing conditions or characteristics of the cannula or the like.

### BRIEF DESCRIPTION OF THE INVENTION

The device for fixing cannulas and the like to the body of a patient as further disclosed in claim 1 is characterised by its rationality as it provides distinct and secure means for holding the cannula or catheter in its functional position, for holding the flexible conduct that feeds the therapeutic fluid to be introduced into the body and protecting the assembly against agents of a mechanical or other nature that hinder the stated function.

To facilitate comprehension, the present description is accompanied by a set of drawings that illustrate by way of a non-limitative example, an embodiment of the device for fixing cannulas and similar instruments to a part of the human body, according to the principles of the claims.

### BRIEF DESCRIPTION OF THE DRAWINGS

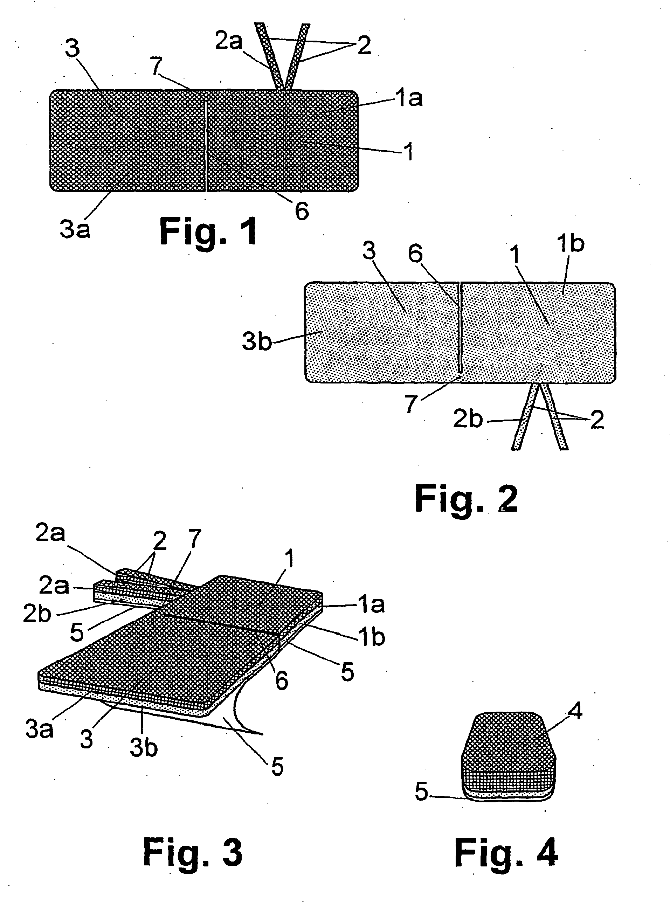

- Fig. 1 and Fig. 2: represent the active upper and lower faces respectively of the new fixing device.
- Fig. 3: is a perspective view of the new device with its lower protective element in the phase of separation.
- Fig. 4: represents a skin protector destined to be placed between the device described herein and the part of the human body in question.

### DETAILED DESCRIPTION OF THE INVENTION

The elements illustrated with numbers in the drawings correspond to the parts indicated below.

The device for fixing cannulas and the like to the human body comprises essentially a band 1 for fixing to the skin of the person in question, a band made of a flexible, extendable and resistant material 1a that is also porous and permeable to air and water vapour.

This material may consist of a nonwoven fabric and its lower face is covered by a layer 1b of hypoallergenic adhesive with suitable properties from the clinical-sanitary point of view.

The band 1 may have any regular geometrical shape, such as rectangular with rounded corners as shown in the drawings. From one side of the band tongue pieces or appendices 2 extend (required for secure fixing) destined for the immediate holding of the cannula or the like, and from another of its sides there is a band 3 for fixing to the cannula grip itself.

The tongue pieces or appendices 2, insofar as they form part of and extend from the band 1, have their lower faces likewise covered by layers 2b of hypoallergenic adhesive.

The band 3 for fixing to the grip of the cannula or the like, is arranged, as mentioned, adjacent to and as an extension of the band 1, which is separated from the latter by a cutting line 6 of a slightly shorter length than the width of both bands, defining a narrow zone 7 that can be easily torn when it is convenient to separate the two bands 1 and 3.

The above mentioned components of the device are protected prior to use, especially on their lower faces covered by the adhesive 1b, 2b, 3b, by a sheet 5 of laminar material with paraffin or plastic, which can be easily separated prior to applying the fixing bands and the solidly joined tongue pieces.

The device is completed with a sheet 4 for protecting the skin of the body part in question, a sheet destined to be placed between the device and the skin, and made of hypoallergenic material and covered temporarily on its lower face by another separable protection sheet 5.

Optionally, the band 1 for fixing to the skin could be provided with a transparent area that would make it possible to observe the state of the skin in the area where the device is applied, to check for example for reddening of the skin, venous inflammation (phlebitis) or a similar anomaly.

## Claims

1. Device for fixing cannulas and the like to a part of the human body, for securing in a temporarily unmovable position an element for therapeutic transfusion using bloodless techniques, comprising a band (1) for fixing to the skin, tongue pieces (2) extending from the band (1) for the immediate holding of the cannula or the like, a second band (3) for fixing to the grip of the cannula or the like, and a skin protector (4) destined to be placed between the device and the human body part in question, wherein the second band (3) for fixing to the grip of the cannula or the like is arranged adjacent to and as an extension of the band (1), **characterised in that** the second band (3) is separated from the band (1) by a cutting line (6) suitable for separating it by tearing a zone (7) and is made of the same material (3a) as the band (1) and is likewise covered on its lower face by a layer (3b) of hypoallergenic adhesive.

2. Device for fixing cannulas and the like to a part of the human body, according to claim 1, **characterised in that** the band (1) for fixing to the skin is made of a material (1a) that is flexible, extendable and resistant, as well as porous and permeable to air and water vapour, such as a nonwoven fabric, covered on its lower face by a layer (1 b) of hypoallergenic adhesive.

3. Device for fixing cannulas and the like to a part of the human body, according to claim 1, **characterised in that** the tongue pieces (2) for holding the grip of the cannula or the like are solidly joined to and made of the same material (2a) as the band (1) and are likewise covered on their lower faces by respective layers (2b) of hypoallergenic adhesive.

4. Device for fixing cannulas and the like to a part of the human body, according to claim 1, **characterised in that** it comprises a sheet (4) for protecting the skin, destined to be placed between the device and the body part in question, made of a hypoallergenic material and covered on its lower face by a separable sheet (5) that is separable immediately prior to its application on the body part in question.

## Patentansprüche

1. Vorrichtung zur Fixierung von Kanülen und dergleichen an einem Teil des menschlichen Körpers, um ein Element für eine therapeutische Transfusion unter Verwendung unblutiger Verfahren vorübergehend unbewegbar zu befestigen, mit einem Band (1) zur Befestigung an der Haut, sich von dem Band (1) aus erstreckenden Zungenteilen (2) zum unmittelbaren Halten der Kanüle oder dergleichen, einem zweiten Band (3) zur Befestigung am Griff der Kanüle oder dergleichen, und einem Hautschutz (4), der zur Anordnung zwischen der Vorrichtung und dem betreffenden Teil des menschlichen Körpers vorgesehen ist, wobei das zweite Band (3) zur Befestigung an dem Griff der Kanüle oder dergleichen nahe dem Band (1) und als Verlängerung desselben angeordnet ist, **dadurch gekennzeichnet, dass** das zweite Band (3) von dem Band (1) durch eine Schnittlinie (6) getrennt ist, welche zum Abtrennen desselben durch Zerreißen eines Bereichs (7) geeignet ist und aus dem selben Material (3a) besteht wie das Band (1) und ebenfalls auf seiner Unterseite mit einer Schicht (3b) hypoallergenen Klebers bedeckt ist.

2. Vorrichtung zur Fixierung von Kanülen und dergleichen an einem Teil des menschlichen Körpers nach Anspruch 1, **dadurch gekennzeichnet, dass** das zur Befestigung auf der Haut vorgesehene Band (1) aus einem Material (1a) besteht, das sowohl flexibel, dehnbar und widerstandsfähig, als auch porös und durchlässig für Luft und Wasserdampf ist, beispielsweise ein Vlies, das auf seiner Unterseite mit einer Schicht (1b) hypoallergenen Klebers bedeckt ist.

3. Vorrichtung zur Fixierung von Kanülen und dergleichen an einem Teil des menschlichen Körpers nach Anspruch 1, **dadurch gekennzeichnet, dass** die Zungenteile (2) zum Halten des Griffs der Kanüle oder dergleichen, fest mit dem Band (1) verbunden sind und aus dem selben Material bestehen wie dieses und ebenfalls auf ihren Unterseiten mit jeweiligen Schichten (2a) hypoallergenen Klebers bedeckt sind.

4. Vorrichtung zur Fixierung von Kanülen und dergleichen an einem Teil des menschlichen Körpers nach Anspruch 1, **dadurch gekennzeichnet, dass** sie eine Bahn (4) für den Schutz der Haut aufweist, die zum Platzieren zwischen der Vorrichtung und dem betreffenden Körperteil vorgesehen ist, aus einem hypoallergenen Material besteht und auf ihrer Unterseite mit einer lösbaren Bahn (5) bedeckt ist, die zum Lösen unmittelbar vor dem Aufbringen der Bahn auf dem betreffenden Körperteil vorgesehen ist.

## Revendications

1. Dispositif pour fixer des canules ou moyens analogues à une partie du corps humain, pour fixer dans une position provisoirement inamovible, un élément de transfusion thérapeutique sans utiliser des techniques intraveineuses comprenant :
- une bande (1) pour être fixée à la peau,
- des pièces en forme de languettes (2) issues de la bande (1) pour tenir immédiatement la canule ou moyen analogue,
- une seconde bande (3) pour fixer à la poignée de la canule ou analogue, et
- un protecteur de peau (4) destiné à être placé entre le dispositif et la partie du corps humain concernée,
dispositif dans lequel
la seconde bande (3) destinée à être fixée à la poignée de la canule ou moyen analogue est adjacente à la bande (1) ou dans le prolongement de celle-ci,
dispositif **caractérisé en ce que**
la seconde bande (3) est séparée de la bande (1) par une ligne de coupe (6) permettant de la séparer en déchirant une zone (7) et elle est réalisée dans la même matière (3a) que la bande (1) et elle est couverte de même manière sur sa face inférieure par une couche (3b) d'un adhésif hypoallergène.

2. Dispositif de fixation de canules ou analogues à une partie du corps humain selon la revendication 1,
**caractérisé en ce que**
la bande (1) pour être fixée à la peau est réalisée en un matériau (1a) souple, extensible et résistant aussi bien que poreux et perméable à l'air et à la vapeur d'eau tel qu'un non-tissé dont la face inférieure est couverte par une couche (1b) d'adhésif hypoallergène.

3. Dispositif de fixation de canules ou analogues à une partie du corps humain selon la revendication 1,
**caractérisé en ce que**
des pièces en forme de languettes (2) pour tenir la poignée de la canule ou analogue, sont réunies solidairement à la bande (1), sont réalisées dans la même matière (2a) et sont couvertes de la même manière sur leur face inférieure par des couches respectives (2b) d'adhésif hypoallergène.

4. Dispositif de fixation de canules ou analogues à une partie du corps humain selon la revendication 1,
**caractérisé en ce qu'**
il comporte une feuille (4) pour protéger la peau, cette feuille destinée à être placée entre le dispositif et la partie du corps humain concernée, étant réalisée en un matériau hypoallergène et elle est couverte sur sa face inférieure d'un film séparable (5) qui est enlevé immédiatement avant son application sur la partie du corps concernée.
